# EUROPEAN PATENT APPLICATION

(11) **EP 2 356 984 A1**
(43) Date of publication of application: **17.08.2011**
(21) Application number: 10152676.2
(22) Date of filing: 04.02.2010
(51) Int. Cl.: A61K 9/20, A61K 31/4425

(54) **Improved distigmine bromide formulation**

(71) Applicant: Nycomed Austria GmbH, 4020 Linz (AT)
(72) Inventor: Ruckendorfer, Hermann, A-4193, Reichenthal (AT); Schweighofer, Rudolf, A-4020, Linz (AT)
(74) Representative: Johansen, Marianne

(57) **Abstract**

The present invention relates to a composition comprising
i) distigmine bromide,
ii) microcrystalline cellulose, and
iii) one or more adsorbent agents.

## Description

### Field of the invention

The present invention provides an improved distigmine bromide formulation, notably in tablet form. It has been found that the tablet composition presently on the market has several disadvantages, especially with respect to stability.

### Background

Distigmine bromide (DB) is a cholinergic drug (indications: urinary incontinence, retention of urine, post operative paralysis of the small bowel, Myasthenia gravis). Distigmine bromide is a rather complicated active ingredient for further processing based upon its high hygroscopicity and interactions with environmental humidity at even short time exposure to ambient room temperature conditions.

This invention was triggered by numerous challenges with standard distigmine bromide tablets (Ubretid®) on European and overseas markets (e.g. Japan). Challenges reported on standard DB tablets refer to manufacturing, subdivision and stability issues. All these standard DB tablet formulations employ various types of starch binders and the manufacturing method involves time consuming wet granulation processes. In combination with the relatively low dosed 5 mg DB as active ingredient these formulations often exert hardness problems ("soft" tablets) combined with inaccurate subdivision at the rather "flat" tablet score of Ubretid® tablets (i.e. the marketed tablets containing DB). Based on the therapeutical indications of DB (see above) and the rather narrow "therapeutic window" of this highly potent drug, inhomogeneous breakability of the DB tablets could lead to safety issues, described as cholinergic crisis in the scientific literature.

Accordingly, there is a need for developing an improved DB formulation that eliminates or reduces the above-mentioned technical problems seen with the present formulation.

### Description of the invention

The present invention provides an improved distigmine bromide formulation. The shortcomings of standard distigmine bromide (Ubretid®) tablets currently on the market can be summarized as follows:
- Even though a rather robust standard type starch formulation has been chosen initially, DB was found to significantly reduce the hardness of the tablets compared to the same formulation without DB (see also comments to Example 2 herein). Apparently water based granulation processes with starch as binder is not optimal for compounds like DB from the processing point of view.
- Furthermore, the present inventors have found that time and labor intensive wet granulation using starch type binders with subsequent drying do not seem to lead to a formulation having favorable characteristics with respect to technical and/or stability properties. Moreover, a wet granulation process is costly and, accordingly, not preferable from an economical point of view.
- Usually in case of DB tablets only a very low hardness (often below 40 Newton) can be reached at the compressing step resulting in "soft" tablets, which are difficult to handle in respect of packaging (e.g. into blisters), due to their lack of hardness and/or their friability. Moreover, such tablets have been found not to have an accurate breakability (e.g. at the tablet score); this is generally a problem for such "soft" and small tablets (only 9 mm in diameter). In addition the rather "flat" score of current Ubretid® tablets impairs the breakability further.
- Attempts to press DB tablets harder (e.g. close to 40 Newton) - if at all possible - often impacts dissolution rates.
- Modifications of the original corn starch formulation (Example 1) led to implementation of pregelatinized corn starch and reduction of Magnesium Stearate (Example 2).
- New stability investigations with DB standard tablets led to shelf life reductions from e.g. 5 years to 3 or even 2 years in several countries, i.e. the tablets were not as stable as originally expected.
- In general a lack of robustness in terms of high batch to batch variability could be observed with the starch type DB formulation when using a wet granulation process.

The present invention solves or reduces the above-mentioned problems seen with the current distigmine bromide formulation by providing a composition comprising
i) distigmine bromide,
ii) microcrystalline cellulose, and
iii) one or more adsorbent agents.

Normally, the concentration of distigmine bromide in a composition of the present invention is from about 1 to about 10% w/w such as from about 1.5% to about 5% w/w.

As mentioned above, distigmine bromide is a very hygroscopic agent and, accordingly, it could be assumed that adding an agent that is water-repellant would improve the formulation. However, the present inventors have surprisingly found that use of an adsorbent agent that in fact is hygroscopic, i.e. is able to adsorb water, in a distigmine bromide formulation leads to a desired result, namely a formulation with improved properties with respect to stability (e.g. with respect to content of unwanted impurities/degradation products, and with respect to stability of dissolution properties) and handling characteristics (e.g. friability, hardness, breakability etc.). A suitable adsorbent agent is an agent, wherein the equilibrium moisture at 65% relative humidity is from about 2 to about 10% w/w and/or at 100% relative humidity is at least 50% w/w such as at least 80% w/w or about 100% w/w.

As demonstrated in the examples herein, an example of such an adsorbent agent is an agent that comprises silicon dioxide such as colloidal silicon dioxide. Notably, the adsorbent agent is a silicon dioxide. However, once the idea of improving the distigmine bromide formulation is understood by a person skilled in the art, he may identify other materials having adsorbent properties such as e.g. mixtures of microcrystalline cellulose and silicon dioxide, silicified microcrystalline cellulose, silicates, aluminum silicates etc. Although at present not tested, it is expected that use of such other materials in a distigmine bromide formulation also may lead to improved results compared with the current marketed tablet formulation.

The adsorbent agent(s) is/are present in the formulation in a total concentration of from about 0.1 % w/w to about 4 % w/w. More specifically, the concentration may be from about 0.1 % w/w to about 1 % w/w, from about 0.2% w/w to about 1 % w/w or from about 0.2% to about 0.8% w/w.

In order to avoid a process involving wet granulation, the presence of microcrystalline cellulose is important. Microcrystalline cellulose has many functions and is widely used in the pharmaceutical industry as e.g. a binder, a diluent, a disintegrant and it also to a minor extent has lubricating properties. Microcrystalline cellulose is commercially available in many different grades that differ with respect to their manufacturing method. Moreover, the source of the cellulose may be different; it may be from a seed or a non-seed source. The various grades may also have different mean particle sizes and co-processed mixtures of microcrystalline cellulose with other pharmaceutically acceptable excipients may also be suitable for use in the present invention (see e.g. Handbook of Pharmaceutical Excipients, 4th Edition, PhP Pharmaceutical Press, pages 108-111), which hereby is incorporated by reference.

The concentration of microcrystalline cellulose in the composition of the invention is normally from about 20 to about 50% w/w. More specifically, the concentration may be from about 25% to about 40% w/w.

A composition of the present invention may also comprise one or more other pharmaceutically acceptable excipient(s). In a separate paragraph in the text below excipients normally used in the preparation of solid dosage form are mentioned. It is envisaged that such excipients may also be present in a composition of the invention. If present, a person skilled in the art will know that the nature of the excipient and the concentration of the excipient used should not counteract the positive effects of the presence of microcrystalline cellulose and the adsorbent agent.

In general, a typically excipient suitable for use in a composition of the invention is e.g. a pharmaceutically acceptable filler like e.g. lactose. As discussed above in the "Background of the invention" section the current marketed tablet formulation contains corn starch and the present inventors believe that at least some of the negative effects identified for the current formulation can be ascribed to this ingredient. Accordingly, it is preferred that maize starch is not present in a composition of the invention, or at the most is present in a concentration that does not have a negative impact on the stability and technical properties of the composition. The upper limit therefore has currently not been identified, but is expected to be at the most about 10% w/w, more likely at the most 2-5% w/w. It is envisaged that other starches may have the same negative impact on the formulation and, accordingly, it is contemplated that such starches, if present, should only be present in an amount that do not have any substantial negative effect on the formulation. It is envisaged that the concentrations mentioned above for maize starch also apply for other kinds of starches.

In those cases, where one or more pharmaceutically acceptable fillers are present in a composition of the invention, it is normally present in a total concentration of from about 50 to about 77% w/w.

Another type of pharmaceutically acceptable excipients that may be present in a composition of the invention is a lubricant. The lubricant is normally selected from magnesium stearate, calcium stearate, stearic acid, or talc. The function of the lubricant is to avoid adherence to the manufacturing apparatus and/or to improve flowability of the solid composition in order to obtain a correct dosing.

The total concentration of lubricant(s) in the composition is generally from about 2 to about 15% w/w such as from about 3 to about 10% w/w.

A composition of the present invention is a solid composition. Thus, it may be in the form of a powder, a granulate or it may be present in a solid dosage form such as a tablet, a capsule, a lozenge, a sachet or the like. Preferably, the composition is in the form of a tablet. The tablet may be coated e.g. with a film-coating such as those known by a person skilled in the art.

As mentioned herein before,technical problems identified for the current tablet formulation are the lack of robustness at manufacture (e.g. hardness), properties of the tablet (eg breakability) and the lack of long-term stability, particularly under hot/humid climate conditions (eg under a tropical zone characterised by 30°C/ 65- 70 % RH). Accordingly, the aims when developing the present composition were to develop a composition with an improved robustness, improved tablet properties and better stability compared with the current tablet formulation on the market. Improved robustness and better stability may be with respect to one or more of the following:
i) improved hardness,
ii) improved breakability,
iii) improved dissolution stability, and
iv) improved stability with respect to chemical degradation of distigmine bromide.

With respect to i) improved hardness this means that tablets can be compressed at a hardness of above 40 Newton, which represents a challenge for the current tablet formulation. Moreover, in the event that the tablets are designed to be divided into e.g. two halves, the hardness of the tablets should meet the requirements of 1) a sufficient hardness for handling of the tablets and 2) a sufficient ability to divide the tablet into two halves.

With respect to the above ii) improved breakability is defined as the breaking of 30 tablets by hand into two half tablets (along the tablet score) followed by weighing each of the 60 parts individually, and calculating the average mass of the tablet halves. The relative standard deviation should not exceed 10 %.

With respect to the above iii) improved dissolution stability it is meant that the dissolution does not significantly change during the shelf-life of the composition. Based on the currently available stability data from the new formulation, an extended shelf-life of 5 years at storage at 25 °C and 60 % RH (as defined/recommended in ICH Q1A Guideline for long term stability testing) seems to be possible. A suitable short term measure is that the average total amount of distigmine bromide released from 6 samples of a composition stored for 6 months at 40°C and 75 % RH (as defined/recommended in ICH Q1A Guideline for accelerated stability testing) is not less than 93 % of the initial average total amount of distigmine bromide released from 6 samples prior to storage at 40°C and 75 % RH. The release is measured by testing the samples according to the Dissolution Method, Ph. Eur. Paddle method, 50 rpm, 37 °C, test medium: 0.1 N hydrochloric acid for 30 min. Normally, such a stability test is initiated at the most 1 month after the manufacturing of the composition. Moreover, a composition of the present invention is expected to fulfill the above-mentioned test during the extended shelf life.

With respect to iv) improved stability with respect to chemical degradation of distigmine bromide, a suitable measure is that the content of 3-HPBM at the most is 0.05% w/w, such as at the most is 0.04% w/w, or 0.03% w/w based on the weight of the composition after storage for 6 months at 40°C and 75% RH in glass containers with a plastic cap closure.

### Specific compositions of the invention include:

A composition comprising
i) distigmine bromide in a concentration of from about 1 to about 10% w/w, such as from about 1.5% to about 5% w/w.
ii) microcrystalline cellulose in a concentration of from about 20 to about 50% w/w such as from about 25% to about 40% w/w, and
iii) one or more adsorbent agents in a concentration of from about 0.1 % to about 4 % w/w such as from about 0.2% to about 1% w/w.

A composition comprising
i) distigmine bromide in a concentration of from about 1 to about 10% w/w such as from about 1.5% to about 5% w/w.
ii) microcrystalline cellulose in a concentration of from about 20 to about 50% w/w such as from about 25% to about 40% w/w, and
iii) one or more adsorbent agents in a concentration of from about 0.1 % to about 4 % w/w such as from about 0.2% to about 1% w/w,
iv) a filler in a concentration of from about 50 to about 77% w/w.

A composition comprising
i) distigmine bromide in a concentration of from about 1 to about 10% w/w such as from about 1.5% to about 5% w/w.
ii) microcrystalline cellulose in a concentration of from about 20 to about 50% w/w such as from about 25% to about 40% w/w, and
iii) one or more adsorbent agents in a concentration of from about 0.1 % to about 4 % w/w such as from about 0.2% to about 1% w/w,
iv) a filler in a concentration of from about 50 to about 77% w/w,
v) a lubricant in a concentration of from about 2 to about 15% w/w such as from about 3 to about 10% w/w.

A composition comprising
i) distigmine bromide in a concentration of from about 1 to about 10% w/w such as from about 1.5% to about 5% w/w.
ii) microcrystalline cellulose in a concentration of from about 20 to about 50% w/w such as from about 25% to about 40% w/w, and
iii) a silicon dioxide in a concentration of from about 0.1 % to about 4 % w/w such as from about 0.2% to about 1% w/w.

A composition comprising
i) distigmine bromide in a concentration of from about 1 to about 10% w/w such as from about 1.5% to about 5% w/w.
ii) microcrystalline cellulose in a concentration of from about 20 to about 50% w/w such as from about 25% to about 40% w/w, and
iii) a silicon dioxide in a concentration of from about 0.1 % to about 4 % w/w such as from about 0.2% to about 1% w/w,
iv) a filler in a concentration of from about 50 to about 77% w/w.

A composition comprising
i) distigmine bromide in a concentration of from about 1 to about 10% w/w such as from about 1.5% to about 5% w/w.
ii) microcrystalline cellulose in a concentration of from about 20 to about 50% w/w such as from about 25% to about 40% w/w, and
iii) a silicon dioxide in a concentration of from about 0.1 % to about 4 % w/w such as from about 0.2% to about 1% w/w,
iv) lactose in a concentration of from about 50 to about 77% w/w.

A composition comprising
i) distigmine bromide in a concentration of from about 1 to about 10% w/w such as from about 1.5% to about 5% w/w.
ii) microcrystalline cellulose in a concentration of from about 20 to about 50% w/w such as from about 25% to about 40% w/w, and
iii) a silicon dioxide in a concentration of from about 0.1 % to about 4 % w/w such as from about 0.2% to about 1% w/w,
iv) a filler in a concentration of from about 50 to about 77% w/w,
v) a lubricant in a concentration of from about 2 to about 15% w/w such as from about 3 to about 10% w/w.

A composition comprising
i) distigmine bromide in a concentration of from about 1 to about 10% w/w such as from about 1.5% to about 5% w/w.
ii) microcrystalline cellulose in a concentration of from about 20 to about 50% w/w such as from about 25% to about 40% w/w, and
iii) a silicon dioxide in a concentration of from about 0.1 % to about 4 % w/w such as from about 0.2% to about 1% w/w,
iv) lactose in a concentration of from about 50 to about 77% w/w,
v) a lubricant in a concentration of from about 2 to about 15% w/w such as from about 3 to about 10% w/w.

A composition comprising
i) distigmine bromide in a concentration of from about 1 to about 10% w/w such as from about 1.5% to about 5% w/w.
ii) microcrystalline cellulose in a concentration of from about 20 to about 50% w/w such as from about 25% to about 40% w/w, and
iii) a silicon dioxide in a concentration of from about 0.1 % to about 4 % w/w such as from about 0.2% to about 1% w/w,
iv) lactose in a concentration of from about 50 to about 77% w/w,
v) a mixture of magnesium stearate and talc in a total concentration of from about 2 to about 15% w/w such as from about 3 to about 10% w/w.

A composition comprising:
i) distigmine bromide in a concentration of from about 1 to about 3% w/w,
ii) microcrystalline cellulose in a concentration of from about 25 to about 30% w/w,
iii) a silicon dioxide in a concentration of from about 0.2 to about 0.6% w/w,
iv) lactose in a concentration of from about 60 to about 70% w/w,
v) talc in a concentration of from about 1 to about 5% w/w,
vi) magnesium stearate in a concentration of from about 0.5 to about 2.5% w/w

A composition comprising:
i) distigmine bromide in a concentration of 2% w/w
ii) microcrystalline cellulose in a concentration of about 25 % w/w,
iii) a silicon dioxide in a concentration of about 0.4 % w/w,
iv) lactose in a concentration of from about 66% w/w,
v) talc in a concentration of about 2.4% w/w,
vi) magnesium stearate in a concentration of about 1.2% w/w.

As demonstrated in the examples a suitable quality of a silicon dioxide is colloidal silicon dioxide.

In another aspect, the invention relates to a method for preparing a composition as described above. However, this turned out to be quite challenging as the lack of sufficient robustness, insufficient breakability and stability problems observed with the DB tablets on the market were not easily overcome and a development program was initiated to improve the formulation, the manufacturing process and/or the design/tablet shape of DB 5 mg tablets. Even development of formulations using modern direct compression excipient mixtures (containing cellulose derivatives, lactose, lubricants, etc) turned out to be very challenging, namely:
- DB particle size is different from excipient particle size like lactose, cellulose - thus homogenous distribution of the low dosed API was a challenge, which was finally solved by the pre-sieving of DB and an adsorbent agent (e.g. silicon dioxide). This also solved other hurdles ("sticky" phenomenon).
- DB obtained by using the usual direct compression excipient mixtures (e.g. see Example 3) led to "sticky"/adhesiveness effects of tablets and compression tools. This was particularly the case at up-scaling trials on high speed rotary presses - thus further optimizations of the nature and concentration of excipients and/or processing parameters were necessary.
- Increased additions of the adsorbent agent (e.g. colloidal silicon dioxide) reduced the "sticky" effect; however, it negatively impacted content uniformity (see Example 4).
- Lack of robustness in general, including surprisingly large differences in small and high scale manufacture with standard excipient mixtures even though the API was rather low dosed (2 %) and relatively simple direct compression methods were applied.

Thus, a preferred method for manufacturing a composition comprising distigmine bromide of the present invention comprises
i) admixing distigmine bromide and one or more adsorbing agents,
ii) pre-sieving the thus obtained mixture, and
iii) adding microcrystalline cellulose and, optionally, one or more pharmaceutically acceptable excipients.

The thus obtained mixture may then be subjecting to a step of compressing the mixture obtained in step iii), i.e. by direct compression. In general, it is envisaged that a wet-granulation step comprising addition of a binder in an aqueous medium should be avoided due to the hygroscopic properties of distigmine bromide. However, other processes involving a granulation step, i.e. roller compaction or agglomeration with ethanol may be suitable methods as well. As demonstrated in the examples herein, a suitable method involves intimate mixing of the ingredients followed by direct compression.

In detail, a method for manufacturing a composition of the present invention comprises pre-sieving the distigmine bromide together with one or more adsorbing agents in order to ensure an even distribution of digstimine bromide on the adsorbing agent and moreover this process is contemplated to impart a certain protection of distigmine bromide towards external humidity (distigmine bromide is hygroscopic). Accordingly, other processes than pre-sieving that lead to the same result are also expected to be suitable for use in the preparation of a composition of the present invention. The pre-sieving is normally conducted by use of a sieve (0.1-0.5 mm, notably - as demonstrated in the examples herein - 0.25 mm).

After such a pre-sieving step a small amount of a suitable filler (e.g. 10-50%) may optionally be added and optionally this mixture is sieved again. As demonstrated in the examples herein a suitable filler is lactose, and about 10-50% may be added. Also as demonstrated in the examples, this pre-mix can be sieved before the remaining ingredients including microcrystalline cellulose are added. Tablets are compressed from the final mixture.

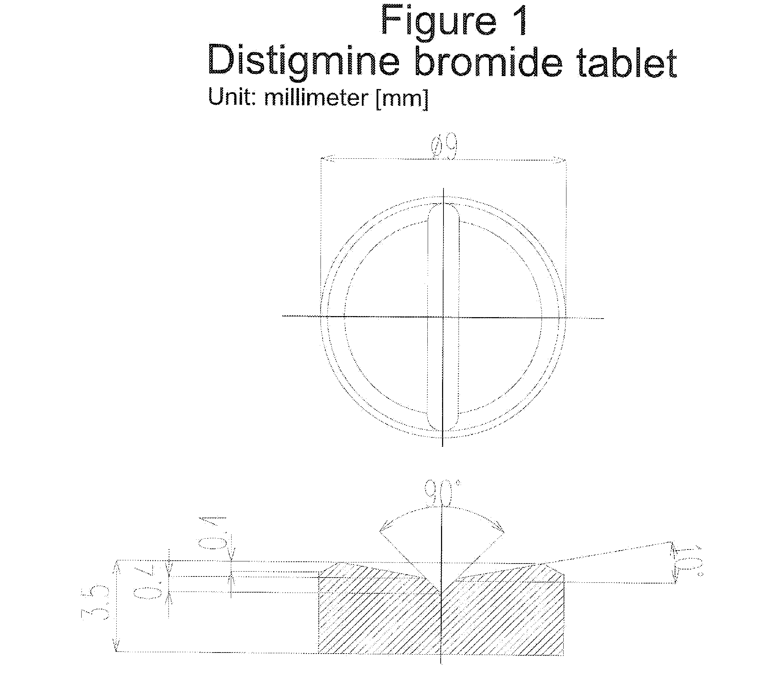

As mentioned herein before, such tablets may be designed to be broken into e.g. two halves or four quarters. In such situations it is very important that the halves or quarters, respectively, comprise the identical amount of active ingredient (distigmine bromide) in order to avoid any over- or under dosing. In Figure 1 herein is shown a suitable form of a tablet that is designed to be broken into two halves.

The details and particulars mentioned above under the composition aspect apply *mutatis mutandis* to the method aspect (e.g. with respect to ingredients, concentration, stability etc.).

As mentioned herein before, one of the problems observed with the current marketed tablet formulation was the variability with respect to breakability. To this end, the present inventors have found a suitable shape for a composition of the present invention in tablet form. The form is depicted in Figure 1. A composition according to the invention in the form of a tablet having the form depicted in Figure 1 can be broken into two parts (tablet halves) and the relative standard deviation of the average mass of the tablet halves should not exceed 10 %.

For detailed descriptions of the manufacturing processes see the examples herein.

*Pharmaceutically acceptable excipients:* In the present context, the term "pharmaceutically acceptable excipient" is intended to denote any material, which is inert in the sense that it substantially does not have any therapeutic and/or prophylactic effect per se. A pharmaceutically acceptable excipient may be added to the active drug substance with the purpose of making it possible to obtain a pharmaceutical composition, which has acceptable technical properties. Although a pharmaceutically acceptable excipient may have some influence on the release of the active drug substance, materials useful for obtaining modified release are not included in this definition.

As mentioned above, apart from the active substance, two substances are mandatory in a composition of the present invention, namely microcrystalline cellulose and an adsorbent agent. Other pharmaceutically acceptable excipients may also be added in order to e.g. improve the ability to manufacture the composition or to further improve the technical properties of the composition. The nature and concentration of the individual excipients depend *inter alia* on the specific type and size (e.g. tablet size) of formulation chosen.

Such excipients include those normally used in formulation of solid dosage forms such as, e.g. fillers, binders, disintegrants, lubricants, flavouring agents, colouring agents, including sweeteners, pH adjusting agents, stabilizing agents, etc.

Typically, a disintegrant is selected from the group consisting of: croscarmellose sodium (a cross-linked polymer of carboxymethylcellulose sodium), crospovidone, starch NF; polacrilin sodium or potassium and sodium starch glycolate. Those skilled in the art will appreciate that it is desirable for compressible tablets to disintegrate within 30 minutes, more desirable within 10 min, most desirable within 5 min; therefore, the disintegrant used preferably results in the disintegration of the tablet within 30 minutes, more preferable within 10 min, most preferable within 5 min.

Examples of disintegrants that may be used are e.g. cellulose derivatives, including microcrystalline cellulose, low-substituted hydroxypropyl cellulose (e.g. LH 22, LH 21, LH 20, LH 32, LH 31, LH30); starches, including potato starch; croscarmellose sodium (i.e. cross-linked carboxymethylcellulose sodium salt; e.g. Ac-Di-Sol®); alginic acid or alginates; insoluble polyvinylpyrrolidone (e.g. Polyvidon® CL, Polyvidon® CL-M, Kollidon® CL, Polyplasdone® XL, Polyplasdone® XL-1 0); sodium carboxymethyl starch (e.g. Primogel® and Explotab®).

Fillers/diluents/binders may be incorporated such as polyols, sucrose, sorbitol, mannitol, Erythritol®, Tagatose®, lactose (e.g., spray-dried lactose, α-lactose, β-lactose, Tabletose®, various grades of Pharmatose®, Microtose or Fast-Floc®), microcrystalline cellulose (e.g., various grades of Avicel®, such as Avicel® PH101, Avicel® PH102 or Avicel® PH105, Elcema® P100, Emcocel®, Vivacel®, Ming Tai® and Solka-Floc®), hydroxypropylcellulose, L-hydroxypropylcellulose (low-substituted) (e.g. L-HPC-CH31, L-HPC-LH11, LH 22, LH 21, LH 20, LH 32, LH 31, LH30), dextrins, maltodextrins (e.g. Lodex® 5 and Lodex® 10), starches or modified starches (including potato starch, maize starch and rice starch), sodium chloride, sodium phosphate, calcium sulfate, calcium carbonate.

In pharmaceutical compositions made according to the present invention, especially microcrystalline cellulose, L-hydroxypropylcellulose, dextrins, maltodextrins, starches and modified starches have proved to be well suited.

More specifically, examples of binders suitable for use in direct compression include cellulose derivates including methylcellulose, hydroxypropylcellulose (HPC, L-HPC), hydroxypropylmethylcellulose (HPMC), microcrystalline cellulose (MCC), sodium carboxymethylcellulose (Na-CMC), etc.;

mono- di-, oligo-, polysaccharides including dextrose, fructose, glucose, isomalt, lactose, maltose, sucrose, tagatose, trehalose, inulin and maltodextrin;

polyols including sugar alcohols such as, e.g, lactitol, maltitol, mannitol, sorbitol, xylitol and inositol;

polyvinylpyrrolidone including Kollidon K30, Kollidon 90F or Kollidon VA64 and

proteins including casein.

Surfactants may be employed such as non-ionic (e.g., polysorbate 20, polysorbate 21, polysorbate 40, polysorbate 60, polysorbate 61, polysorbate 65, polysorbate 80, polysorbate 81, polysorbate 85, polysorbate 120, sorbitane monoisostearate, sorbitanmonolaurate, sorbitan monopalmitate, sorbitan monostearate, sorbitan monooleate, sorbitan sesquioleate, sorbitan trioleate, glyceryl monooleate and polyvinylalkohol), anionic (e.g., docusate sodium and sodium lauryl sulphate) and cationic (e.g., benzalkonium chloride, benzethonium chloride and cetrimide) or mixtures thereof.

Other appropriate pharmaceutically acceptable excipients may include colorants, flavouring agents, and buffering agents.

The compositions may comprise a sweetener selected from the group consisting of dextrose, fructose, glycerin, glucose, lactitol, lactose, maltose, sucrose, tagatose, trehalose, alitame, aspartame, acesulfam potassium, cyclamic acid, cyclamate salt (e.g. calcium cyclamate, sodium cyclamate), sucralose, neohesperidine dihydrochalcone, thaumatin, saccharin, saccharin salt (e.g. ammonium saccharin, calcium saccharin, potassium saccharin, sodium saccharin), and mixtures thereof.

The tablets of the invention may be provided with a coating provided that the coating does not substantially retard the release of the active drug substance from the composition. Typically, a film coating may be employed.

If desired known flavourants and known FD & C colorants can be added to the composition.

A suitable shape of a composition according to the present invention in the form of a tablet is shown in Figure 1 (top and side view of the tablet).

The invention is illustrated in the following examples without limiting the invention thereto.

### Materials

| Ingredient | Quality | Manufacturer/Supplier |
|---|---|---|
| | | |
| Distigmine bromide | In house specification | Nycomed Austria GmbH |
| Magnesium stearate | Ph.Eur. | FACl S.p.A or Magnesia GmbH |
| Talc | Ph.Eur. | Rio Tinto Minerals |
| Maize starch | Ph.Eur. | Cargill |
| Starch, pregelatinised | Ph.Eur. | Cargill |
| Lactose monohydrate | Ph.Eur. | Meggle |
| Microcrystalline Cellulose (Vivapur 12, Avicel PH 102) | Ph.Eur. | J. Rettenmaier & Söhne GmbH & Co KG |
| Colloidal Silicon Dioxide (Aerosil 200) | Ph.Eur. | Evonik Degussa |

### Methods

### Appearance:

A representative amount of units is evaluated visually on a sheet of white paper in diffuse light.

### Diameter, Thickness:

The dimensions of 20 tablets are determined with an accuracy of 0.01 mm using a suitable instrument e.g. slide calliper.

Distigmine bromide - Identification and assay, uniformity of content, purity_(3-Hydroxypyridine methylbromide and Additional related substances)

### HPLC method:

Instrument:_High pressure liquid chromatograph with autosampler, UV-detector and integration unit.
Column: Inertsil C8 (5 µm) 150 x 4.6 mm or equivalent. Injection volume: 25 µl
Mobile phase A: Dissolve 9.0 g NaCl per liter of water (= solvent).
Mobile phase B: Acetonitrile/0.9% NaCl = 80/20 v/v;
Gradient elution: 0-2 min: 100 % mobile phase A; 2- 35 min: linear switch from 100% mobile phase A to 100 % mobile phase B ; 35 - 40 min: return from 100% mobile phase B to 100 % mobile phase A
Flow : 1.5 ml/min; Detection: 0- 4.5 min: UV 320 nm; 4.5- 30 min: UV 270 nm

The content uniformity test is performed according to Ph.Eur. 2.9.6 (Test A)

Distigmine bromide - identification (UV): The UV spectrum between 190 nm and 400 nm is measured, using 0.1 M Hydrochloric acid as compensation liquid.

### Average mass, Uniformity of mass (Ph. Eur.):

Weigh individually 20 units taken at random or, for single-dose preparations presented in individual containers, the contents of 20 units, and determine the average mass. Not more than 2 of the individual masses deviate from the average mass by more than the percentage deviation according to Ph. Eur. 2.9.5 and none deviates by more than twice that percentage.

### Disintegration time (in water)

Disintegration time is determined using Disintegration apparatus A (basket-rack assembly) described in Ph. Eur. 2.9.1. The tablets must disintegrate within 15 minutes.

### Friability

Friability is tested according to the method described in Ph. Eur. 2.9.7. Not more than 1.0% loss on mass is allowed. Tablets are not allowed to be cracked, cleaved or broken.

### Dissolution rate (after 30 minutes, 0.1 M HCl):

For dissolution of the tablets, Apparatus 2 described in Ph.Eur. 2.9.3 is used. Samples are analysed by a high pressure liquid chromatograph (HPLC) with autosampler, UV-detector and integration unit.
Solvent:_Hydrochloric acid 0.1 M; Injection Volume: 100 µl
Mobile Phase A:_Sodium chloride 0.9%/ Mobile Phase B:_Acetonitrile: 90/10 vv
Column: PRP1, ID: 4.6 mm, length: 150 mm.
Detection: UV 272 nm

Water content (of granulate and direct compression mixture): Karl Fischer

The water content is determined according to the Karl Fischer Method described in Ph. Eur 2.5.12. The semi-micro determination of water is based upon the quantitative reaction of water with sulphur dioxide and iodine in a suitable anhydrous medium in the presence of a base with sufficient buffering capacity.

Hardness (as IPC during compression)

This test is intended to determine, under defined conditions, the resistance to crushing of tablets, measured by the force (unit: Newton) needed to disrupt them by crushing (Ph. Eur. 2.9.8).

### Breakability

Break 30 tablets by hand into 2 halve tablets (alongside the score) and weigh each of the 60 parts individually and calculate the average mass of the tablet halves. The relative standard deviation should not exceed 10 %.

### Examples

### Example 1 (comparison)

Original Standard tablet formulation for distigmine bromide (DB) (batch No 211251)

| | |
|---|---|
| Distigmine bromide | 5.0 mg |
| Lactose | 151.0 mg |
| Maize starch | 81.0 mg |
| Magnesium Stearate | 5.0 mg |
| Talc | 8.0 mg |
| | 250.0 mg |

Manufacturing process (basic process: wet granulation):
A starch paste is prepared using maize starch and water. In parallel a mixture is prepared with the rest of the maize starch, Distigmine Bromide and lactose. The mixture is granulated with the starch paste, the wet granulate is sieved and dried. The lubricants talc and magnesium stearate are added to the granulate. The obtained final granulate is compressed into tablets on a rotary tablet press.

### Comments

Batch production of this original formulation show several shortcomings in terms of robustness, e.g. batch to batch variability with respect to hardness, dissolution rate profile, etc. Furthermore the stability profile for such tablets (see Example 7) - particularly the content of the degradation product 3 -HPMB - did not support longer shelf lives of e.g. 5 years. Accordingly, there is a need for a new improved formulation.

### Example 2 (comparison)

Modified Standard tablet formulation for DB (batch # 10431365) ― slightly modified starch formulation was developed in order to counteract robustness and stability problems experienced with the original standard tablet formulation.

| | |
|---|---|
| Distigmine bromide | 5.0 mg |
| Lactose | 151.0 mg |
| Maize starch | 77.9 mg |
| Pregelatinized starch | 5.6 mg |
| Magnesium Stearate | 2.5 mg |
| Talc | 8.0 mg |
| | 250.0 mg |

Manufacturing process (basic process: wet granulation):
Lactose monohydrate, maize starch and Distigmine bromide are blended in a high shear mixer. Maize starch is suspended in a small part of purified water. This suspension is added to boiling purified water while stirring. The yielding starch paste is cooled down. The starch paste is added to the active substance mixture under stirring and the resulting granulate is sieved. The granulate is dried in a fluid bed drier and sieved afterwards. Talc, pregelatinised starch and magnesium stearate are added to the dried granulate and mixed in a tumble blender to obtain the final granulate. The final granulate is compressed on a rotary press to tablets.
hardness of tablets (Ph. Eur.): 30 - 40 Newton
friability (Ph Eur): 0.19 %
dissolution rate (Ph. Eur.): 96%
water content: 5.9 %
subdivision: average mass of subdivided tablets (tablet halves) : 124.43 mg ;
relative standard deviation: 10. 68 %

### Comments

Even though robustness parameter like e.g. dissolution rate profiles and batch to batch variability have been improved, a high variation in breakability was seen for this modified standard formulation batch of DB tablets.

Concerning tabletting properties small scale investigations have been performed with such starch type tablet formulations. In small scale trials on excenter tablet presses rather low hardness results of 44 - 49 Newton were observed for such starch type DB tablets. This is quite unexpected since the same kind of tablets without DB (starch type placebo tablets) in small scale trials on excenter tablet presses exhibit very good hardness results of 76- 82 Newton. Thus, it can be concluded that the hardness of the tablet is significantly impaired by the presence of DB, even if DB is only present in a low dose of 5 mg/tablet.

### Example 3

### Directly compressed tablets (F-464041- G)

### Tablet formulation for DB:

| | |
|---|---|
| | |
| Distigmine bromide | 5.0 mg |
| Lactose | 132.0 mg |
| Microcrystalline Cellulose (e.g. Vivapur 12) | 100.0 mg |
| Magnesium Stearate | 5.0 mg |
| Talc | 8.0 mg |
| | 250.0 mg |

Manufacturing process (basic process: direct compression):
Distigmine Bromide was pre-sieved (0.25 mm) and mixed with microcrystalline cellulose and lactose. The lubricants talc and magnesium stearate were added. The mixture was compressed on a rotary press.
hardness of tablets (Ph. Eur.): 50-70 Newton
friability (Ph. Eur): 0.12 %
water content: 4.9 %
dissolution rate (Ph. Eur.): 96 %

Comments: This formulation showed good tabletting properties and very good results in stability results (see example 7) but at upscaling trials they exerted "sticky" effects (tablets glued to compression tools). For further optimization work in terms of excipient ratios and qualities this basic formulation was used. Moreover, this work provides information on the stability.

### Example 4

### Directly compressed tablets (batch V 15050)

Compared to Example 3 the excipient ratio was slightly changed and another type of Microcrystalline Cellulose was used (Avicel PH 102)

| | |
|---|---|
| Distigmine bromide | 5.0 mg |
| Lactose | 122.0 mg |
| Microcrystalline Cellulose (Avicel PH 102) | 100.0 mg |
| Magnesium Stearate | 5.0 mg |
| Talc | 8.0 mg |
| Colloidal Silicon Dioxide | 10.0 mg |
| | 250.0 mg |

Manufacturing process (basic process: direct compression):
Distigmine Bromide was premixed with half of the amount of colloidal silicon dioxide. The rest of the colloidal silicon dioxide was added. After a further mixing step the rest of the excipients were added. The mixture was compressed on a rotary press.

### Comment

This formulation showed acceptable results in terms of tabletting properties in lab scale trials but in upscaling trials the high adsorbent content had a negative impact on the content uniformity of the tablets most likely due to the de-mixing phenomena in the rather voluminous dry mixing step prior to compression. Thus, lubricant/excipient ratio and process had to be adapted further in order to reach an optimized and balanced upscaled formulation (see Example 5)

### Example 5

### Directly compressed tablets (batch 9157)

Further adaptations in the excipient ratios and pre-sieving of the adsorbent/DB admixture led to the final optimized formulation for DB tablets (direct compression method):

| | |
|---|---|
| Distigmine bromide | 5.0 mg |
| Lactose | 165.0 mg |
| Microcrystalline Cellulose (Avicel PH 102) | 70.0 mg |
| Magnesium Stearate | 3.0 mg |
| Talc | 6.0 mg |
| Colloidal Silicon Dioxide | 1.0 mg |
| | 250.0 mg |

Manufacturing process (basic process: direct compression):
Distigmine Bromide was pre-sieved (0.25 mm) and mixed with colloidal silicon dioxide. A small amount of lactose was added. The mixture was sieved again (0.25 mm). Then the rest of the lactose was added, as well as the remaining excipients microcrystalline cellulose, talc and magnesium stearate. The mixture was compressed on a rotary press.

The problem solved with the premixing/presieving of DB with Aerosil is a homogenous DB distribution within the excipient mixture resulting in much better/acceptable content uniformity. It is speculated that the premixing/sieving procedure blocks the adhesive and hygroscopic properties of DB. So an equal DB distribution can be reached via the DB- Aerosil conglomerates by admixture to the main excipients. Aerosil seems to have a neutralizing effect on the hygroscopic and adhesive properties of DB. However the admixture of the voluminous adsorbent colloidal silicon dioxide has certain limitations as Example 4 shows.

Hardness of tablets (Ph. Eur.): 40 - 60 Newton

friability (Ph Eur): 0.17 %

dissolution rate (Ph. Eur.): 96%

water content: 5.0 %

subdivision: average mass of subdivided tablets (tablet halves): 125.68 mg ;

relative standard deviation: 5. 32 %

### Comment

This formulation shows fine results in terms of:
- robustness of manufacture: direct compression, upscaling, no "sticky" effects
- properties of tablets: subdivision, dissolution rate, hardness, content uniformity
- stability trials have been initiated and are ongoing. Good stability profile (see Example 7) is expected.

### Example 6

### Tablet with improved breakability

Composition and manufacture according to Example 5.

Tablet shape with patient friendly score to improve breakability and administration especially for elderly patients:
Tablet tools used for compression:
round, 9 mm (diameter),
lower tablet punch: plain
upper tablet punch: with "Karate" score as depicted in Fig. 1.

### Example 7

### Stability testing

Comparison of stabilities between standard formulation (Example 1) and basic formula of improved formulation (Example 3) for DB tablets after 6 months storage in brown glass bottles with a plastic cap closure:

### a) Stability Profile of standard formulation for DB tablets (batch # 211251)

| Test | Initial Analysis | 6 months at 25C/60 % RH | 6 months at 30C/70 % RH | 6 months at 40C/75 % RH |
|---|---|---|---|---|
| Appearance | white tabl. | white tabl. | white tabl. | white tabl. |
| Average mass | 255 mg | 255 mg | 253 mg | 254 mg |
| Disintegration time^{a} | 5-6 min | 2-6 min | 2-6 min | 2-6 min |
| Friability^{b} | 0.6 % | 0.4 % | 0.4 % | 0.4 % |
| 3-HPMB^{c} | 6 µg/tabl. | 33 µg/tabl. | 44 µg/tabl. | 143 µg/tabl. |
| Other impurities^{d} | n.d. | 0.2 % | 0.2 % | 0.5 %; 0.1 %; 0.2 % |
| Content Distigmine bromide^{e} | 4.97 mg | 4.79 mg | 4.86 mg | 4.56 mg |
| Dissolution rate^{f} | 97 % | 96% | 97 % | 90% |

| | | | | |
|---|---|---|---|---|
| a: determined as described under the heading "Methods" herein b: determined as described under the heading "Methods" herein c: determined as described under the heading "Methods" herein d: determined as described under the heading "Methods" herein e: determined as described under the heading "Methods" herein f: determined as described under the heading "Methods" herein | | | | |

### b) Stability Profile of basic formulation of new improved formulation for DB tablets (batch F-464041- G)

| Test | Initial Analysis | 6 months at 25C/60 % RH | 6 months at 30C/65 % RH | 6 months at 40C/75 % RH |
|---|---|---|---|---|
| Appearance | white tabl. | white tabl. | white tabl. | white tabl. |
| Average mass | 251 mg | 251 mg | 250 mg | 251 mg |
| Disintegration time^{a} | 1-2 min | 1-2 min | 1-2 min | 1-2 min |
| Friability^{b} | 0.1 % | 0.1 % | 0.1 % | 0.1 % |
| 3-HPMB^{c} | 8 µg/tabl. | 7 µg/tabl. | 13 µg/tabl. | 38 µg/tabl. |
| Other impurities^{d} | < 0.1 % | < 0.1 % | < 0.1 %; 0.1 % | < 0.1 %; 0.3 % |
| Content Distigmine bromide^{e} | 4.94 mg | 4.99 mg | 4.92 mg | 4.88 mg |
| Dissolution rate^{f} | 96% | 101 % | 100 % | 97 % |

| | | | | |
|---|---|---|---|---|
| a-f: see above | | | | |

### Comments

A significant increase in terms of stability could be achieved by basic formulation of new improved tablet formulation for DB in terms of
- Content of the stability indicating degradation product: 3- HPMB is substantially lower at all temperature storages, and so is the content of other impurities
- Dissolution rate remains stable even at 6 months "tropical zone" testing (40 °C /75% RH)
- Friability remains at a very low level at all temperatures

### c) Stability Profile of new improved formulation for DB tablets

| Test | Initial Analysis | 3 months at 25C/60 % RH | 6 months at 25C/60 % RH | 6 months at 40C/75 % RH |
|---|---|---|---|---|
| Appearance | | | | |
| Average mass | | | | |
| Disintegration time^{a} | | | | |
| Friability^{b} | | | | |
| 3-HPMB^{c} | | | | |
| Other impurities^{d} | | | | |
| Content Distigmine bromide^{e} | | | | |
| Dissolution rate^{f} | | | | |

| | | | | |
|---|---|---|---|---|
| a-f: see above | | | | |

## Claims

1. A composition comprising
i) distigmine bromide,
ii) microcrystalline cellulose, and
iii) one or more adsorbent agents.

2. A composition according to claim 1, wherein the one or more adsorbent agents are hygroscopic.

3. A composition according to claim 1 or 2, wherein the equilibrium moisture of the one or more adsorbent agents at 65% relative humidity is from about 2 to about 10% w/w and/or at 100% relative humidity is at least 50% w/w, such as at least 80% w/w, or about 100% w/w.

4. A composition according to any one of the preceding claims, wherein the concentration of distigmine bromide in the composition is from about 1 to about 10% w/w such as from about 1.5% to about 5% w/w.

5. A composition according to any one of the preceding claims, wherein the concentration of microcrystalline cellulose in the composition is from about 20 to about 50% w/w such as from about 25% to about 40% w/w.

6. A composition according to any one of the preceding claims, wherein the total concentration of the one or more adsorbent agents is from about 0.1 % to about 4 % w/w, such as from about 0.2% to about 1% w/w.

7. A composition according to any one of the preceding claims further comprising a pharmaceutically acceptable filler.

8. A composition according to claim 7, wherein the filler is lactose.

9. A composition according to claim 8, wherein the pharmaceutically acceptable filler is not maize starch.

10. A composition according to claim 7 or 8, wherein the concentration of the pharmaceutically acceptable filler is from about 50 to about 77% w/w.

11. A composition according to any one of the preceding claims, wherein the one or more adsorbent agents comprises silicon dioxide such as colloidal silicon dioxide.

12. A composition according to any one of the preceding claims, wherein the one or more adsorbent agents is a silicon dioxide.

13. A composition according to any one of the preceding claims further comprising a lubricant.

14. A composition according to claim 13, wherein the lubricant is selected from magnesium stearate, calcium stearate, stearic acid, or talc.

15. A composition according to any one of the preceding claims, wherein the total concentration of lubricant(s) in the composition is from about 2 to about 15% w/w such as from about 3 to about 10% w/w.

16. A composition according to any one of the preceding claims in the form of a tablet.

17. A composition according to any one of the preceding claims, wherein the content of 3-HPBM at the most is 0.05% w/w, such as at the most is 0.04% w/w or 0.03% w/w based on the total weight of the composition after storage for 6 months at 40°C and 75% RH in glass containers with plastic cap closure.

18. A composition according to any one of the preceding claims, wherein the average total amount of distigmine bromide released from 6 samples of a composition stored for 6 months at 40°C and 75 % RH is not less than 93 % of the initial average total amount of distigmine bromide released from 6 samples prior to storage at 40°C and 75 % RH.

19. A method for preparing a composition comprising distigmine bromide, the method comprising
i) admixing distigmine bromide and one or more adsorbing agents,
ii) pre-sieving the thus obtained mixture, and
iii) adding microcrystalline cellulose and, optionally, one or more pharmaceutically acceptable excipients.

20. A method according to claim 19, further comprising a step of compressing the mixture obtained in step iii).

21. A method according to claim 19 or 20, wherein the one or more adsorbing agents is a silicon dioxide.

22. A method according to clam 15, wherein the concentration of silicon dioxide in the composition is from about 0.1 % to about 4 % w/w such as from about 0.2% to about 1 % w/w.

23. A method according to any one of claims 19-23, wherein step iii) includes addition of one or more pharmaceutically acceptable excipients.

24. A method according to claim 23, wherein one or more pharmaceutically acceptable excipients are selected from fillers, binders, disintegrants, lubricants etc.

25. A method according to any one of claims 19-24 for the preparation of a composition as defined in any one of claims 1-18.

26. A composition according to any one of claims 1-19 and/or obtainable by a method according to any one of claims 20-25 in the form of a tablet having a shape essentially as depicted in Figure 1 herein.

27. A composition according to claim 26 that is breakable into sub parts of essentially equal mass, wherein the relative standard deviation around the average mass of the subdivided tablet does not exceed 10 %.

28. A composition according to claim 27, wherein the tablet is breakable into halves or quarters.
